# FASCICULE DE BREVET EUROPEEN

(11) **EP 1 401 432 B1**
(45) Date de publication et mention de la délivrance du brevet: **14.09.2005**
(21) Numéro de dépôt: 02745529.4
(22) Date de dépôt: 14.06.2002
(51) Int. Cl.: A61K 31/415, A61K 31/4164, A61K 31/4174, C07D 233/58, C07D 233/64, A61P 25/08, A61P 9/06, A61P 25/28, A61P 25/24, A61P 25/18, A61P 1/06, A61P 9/10, A61P 29/00

(54) **DERIVES D'IMIDAZOLES MODULANT LES CANAUX SODIQUES**
KALIUMKANAL-MODULIERENDE IMIDAZOLDERIVATE
IMIDAZOLE DERIVATIVES FOR MODULATING SODIUM CHANNELS

(30) Priorité: 15.06.2001 FR 0107820
(43) Date de publication de la demande: 31.03.2004
(73) Titulaire: Société de Conseils de Recherches et d'Applications Scientifiques ( S.C.R.A.S.), 75016 Paris (FR)
(72) Inventeur: BIGG, Dennis, F-91190 Gif-sur-Yvette (FR); POMMIER, Jacques, F-75015 Paris (FR); LIBERATORE, Anne-Marie, F-78610 Auffargis (FR)
(74) Mandataire: Bourgouin, André
(86) Numéro de dépôt international: PCT/FR2002/002039
(87) Numéro de publication internationale: WO 2002/102375

(56) Documents cités:
- WO-A-00/57877
- WO-A-00/71120
- WO-A-01/26656
- WO-A-01/44201
- SUENAGA, K. ET AL: "Catharsitoxins from the Chinese remedy qiung laug" TETRAHEDRON LETTERS (2001), 42(40), 7079-7081, XP004317896
- LIPSHUTZ, BRUCE H. ET AL: "Protection of imidazoles as their [.beta.-(trimethylsilyl)ethoxy]methyl (SEM) derivatives" TETRAHEDRON LETT. (1986), 27(35), 4095-8, XP001053736
- HEERDING, D. A. ET AL: "1,4-Disubstituted imidazoles are potential antibacterial agents functioning as inhibitors of enoyl acyl carrier protein reductase (FabI)" BIOORGANIC & MEDICINAL CHEMISTRY LETTERS (2001), 11(16), 2061-2065, XP001059006
- VENKATARAMAN, B. V. ET AL: "Structure-activity studies of new imidazolines on adrenoceptors of rat aorta and human platelets" NAUNYN-SCHMIEDEBERG'S ARCH. PHARMACOL. (1991), 344(4), 454-63, XP001061258
- "VIDAL" , PARIS * composé XYLOCARD *
- MESTRE M. ET AL.: "Frequency-independent blockade of cardiac Na+ channels by riluzole: comparison with established anticonvulsants and class I anti-arrhythmics" FUNDAM. CLIN. PHARMACOL., vol. 14, 2000, pages 107-117,
- extrait de PUBMED Database accession no. PMID: 1356284 & GRANT AO, WENDT DJ.: "Block and modulation of cardiac Na+ channels by antiarrhythmic drugs, neurotransmittors and hormones" TRENDS PHARMACOL. SCI., vol. 13, no. 9, 1992, pages 352-358,
- extrait de PUBMED Database accession no. PMID: 11273715 & BALSER JR: "The cardiac sodium channel: gating function and molecular pharmacology" J. MOL. CELL. CARDIOL., vol. 33, no. 4, avril 2001 (2001-04), pages 599-613,
- extrait de PUBMED Database accession no. PMID: 9778599 & XIE X, HAGAN RM: "Cellular and molecular actions of lamortrigine: possible mechanisms of efficacy in bipolar disorder" NEUROPSYCHOBIOLOGY, vol. 38, no. 3, 1998, pages 119-130,
- HURLEY S.C.: "Lamortrigine update and its use in mood disorders" THE ANNALS OF PHARMACOTHERAPY, vol. 36, no. 5, 2002, pages 860-873,

## Description

La présente invention concerne de nouveaux dérivés d'imidazoles modulant les canaux sodiques, leur préparation et leurs utilisations thérapeutiques.

Les composés modulateurs de canaux sodiques sont très utiles pour des utilisations thérapeutiques comme:
- le traitement ou la prévention de la douleur, et notamment :
   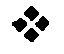 des douleurs neuropathiques telles que la névralgie du trijumeau, les douleurs liées à des maladies virales ou rétrovirales (par exemple les douleurs liées à l'herpès comme la douleur post-herpétique, les douleurs liées au Syndrome Immuno-Déficitaire Acquis (SIDA) ou les douleurs liées au zona), les neuropathies diabétiques, les névralgies glosso-pharyngiennes, les radiculopathies et neuropathies secondaires à des infiltrations métastatiques, l'adiposis dolorosa et les douleurs liées aux brûlures,
   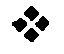 de la migraine,
   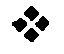 des douleurs post-opératoires,
   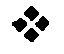 des douleurs centrales consécutives aux accidents cérébraux vasculaires, lésions thalamiques et sclérose en plaques,
   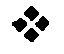 des douleurs chroniques, et
   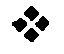 des douleurs liées à un cancer ;
- le traitement de l'épilepsie ;
- le traitement des troubles du rythme cardiaque ;
- le traitement de troubles liés à la neurodégénération, et en particulier :
   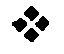 des accidents cérébraux vasculaires,
   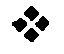 du traumatisme cérébral, et
   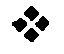 de maladies neurodégénératives telles que la maladie d'Alzheimer, la maladie de Parkinson et la sclérose latérale amyotrophique ;
- le traitement de la dépression et des troubles bipolaires ;
- le traitement du syndrome du colon irritable ;
- le traitement des rétinopathies diabétiques.

Les composés répondant à la formule générale **(I)** telle que définie plus loin sont des modulateurs de canaux sodiques et sont donc susceptibles d'être utilisés pour le traitement des maladies / désordres / troubles cités précédemment.

La demande de brevet PCT WO 95/00493 décrit des intermédiaires de synthèse de formule générale **(A1)** 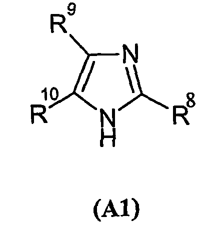 dans laquelle :
R⁸ représente notamment un radical alkyle comptant de 1 à 6 atomes de carbone ou un radical phénylalkyle éventuellement substitué ;
et R⁹ et R¹⁰ représentent notamment indépendamment un atome d'hydrogène, un radical alkyle comptant de 1 à 6 atomes de carbone ou un radical phényle éventuellement substitué par un ou des radicaux choisis notamment parmi un atome halogène et un radical hydroxy, alkyle ou alkoxy.
Toutefois, aucune propriété pharmacologique n'est décrite pour ces composés.

Le brevet US 5,840,721 décrit des agents utiles pour restaurer la sensibilité de cellules cancéreuses résistantes au traitement par des agents de chimiothérapie, lesdits agents répondant à la formule générale **(A2)** 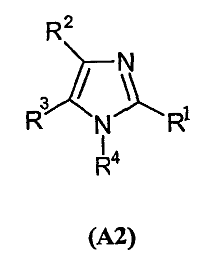 dans laquelle :
R¹ représente notamment un radical aralkyle éventuellement substitué ;
R² et R³ représentent notamment indépendamment un atome d'hydrogène, un radical alkyle comptant de 1 à 6 atomes de carbone ou un radical phényle éventuellement substitué par un ou des radicaux choisis notamment parmi un atome halogène et un radical alkyle ou alkoxy ;
et R⁴ représente notamment un atome d'hydrogène.

La demande de brevet PCT WO 00/57877 décrit des agents bloquant les canaux sodiques, et notamment ceux de formule générale **(A3)** 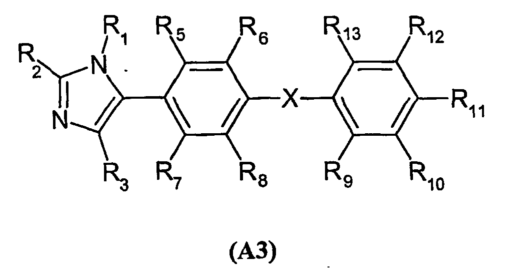 dans laquelle :
R₁ représente notamment un atome d'hydrogène ;
R₂ et R₃ représentent notamment indépendamment un atome d'hydrogène ou un radical alkyle de 1 à 10 atomes de carbone, un radical cycloalkyle ou un radical aryle,
X représente O, S ou un radical NR₁₅ dans lequel R₁₅ représente un atome d'hydrogène, un radical alkyle ou cycloalkyle,
et R₅, R₆, R₇, R₈, R₉, R₁₀, R₁₁, R₁₂ et R₁₃ représentent (notamment) indépendamment un atome d'hydrogène, un atome halogène et un radical alkyle, hydroxy ou alkoxy.

La demande de brevet PCT WO 01/26656 décrit des agents modulant les canaux sodiques, et notamment ceux de formule générale **(A4)** 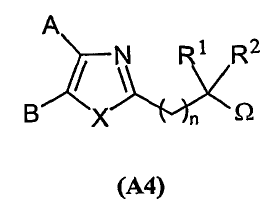 dans laquelle :
A représente (notamment) un radical phényle ou biphényle éventuellement substitué ;
B représente (notamment) un atome d'hydrogène ou un radical alkyle ;
X représente (notamment) NR³⁸, R³⁸ représentant notamment un atome d'hydrogène ou un radical alkyle ;
n est un entier de 0 à 6 ;
Ω représente l'un des radicaux NR⁴⁶R⁴⁷ ou OR⁴⁸ dans lesquels R⁴⁶ et R⁴⁷ représentent (notamment), indépendamment, un atome d'hydrogène ou un radical alkyle, cycloalkyle ou cycloalkylalkyle et R⁴⁸ représente un atome d'hydrogène ou un radical alkyle

Toutefois, comme dans nombre de documents antérieurs présentés dans la demande de brevet PCT WO 00/57877, le motif phénoxyphényle (X représente O) ou phénylthiophényle (X représente S) ou anilinophényle (X représente NR₁₅) semble être l'un des éléments essentiels pour obtenir une activité de modulation des canaux sodiques.

Par ailleurs, la demande de brevet PCT WO 00/71120 décrit des agents à activité antibactérienne qui répondent notamment à la formule générale **(A5)** 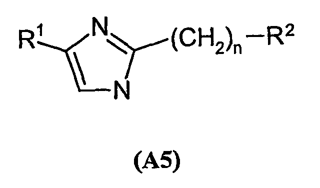 dans laquelle :
R¹ représente un radical C₁₋₄ alkyle, Ar, 2-thiényle ou 3-thiényle ;
R² représente un radical C₁₋₄ alkyle, Ar ;
n est un entier de 0 à 3 ; et
Ar représente phényle ou naphtyle éventuellement substitué de 1 à 3 fois par des substituants tels que C₁₋₄ alkyle, C₁₋₄ alkoxy, CF₃, F, Cl, Br, I, phényle ou méthylènedioxy, lesdits substituants pouvant être obtenus par synthèse chimique et étant stables.

La demande de brevet PCT WO 01/44201 décrit des antagonistes du récepteur Y5 du neuropeptide Y qui répondent notamment à la formule générale **(A6)** 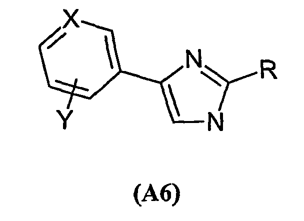 dans laquelle :
R représente (notamment) un radical C₁-C₆ alkyle, C₃-C₇ cycloalkyle, hétérocycloalkyle, hétérocycloalkyl(C₁-C₆)alkyle ou hétéroaryl(C₁-C₆)alkyle ou encore le radical 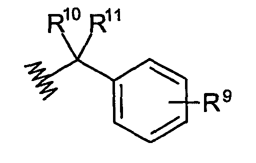
R⁹ représentant de un à trois substituants choisis indépendamment parmi un atome d'hydrogène, un atome halogène et un radical C₁-C₆ alkyle ou C₁-C₆ alkoxy, et R¹⁰ et
R¹¹ étant (notamment) choisis indépendamment parmi un atome d'hydrogène et un radical C₁-C₆ alkyle ;
X représente =CH- ou =N- ; et
Y représente de un à trois substituants choisis indépendamment parmi un atome d'hydrogène, un atome halogène ou un radical trihaloalkyle, C₁-C₆ alkyle, C₁-C₆ alkényle, C₃-C₇ cycloalkyle, C₁-C₆ alkyle substitué par C₃-C₇ cycloalkyle, -OH, -O(C₁-C₆)alkyle, -SH, -S(C₁-C₆)alkyle, ou -CN.

Enfin, un article de Suenaga et coll. (*Tetrahedron Letters* (2001), **42,** 7079-81) décrit les catharsitoxines D et E, c'est-à-dire respectivement le 2-(2-méthylpropyl)-5-phénylimidazole et le 2-(1-méthylpropyl)-5-phénylimidazole. Les propriétés biologiques de ces toxines devaient toutefois encore être étudiées.

Selon l'invention, les composés de formule générale **(I)** 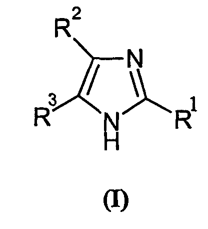 dans laquelle
R¹ représente un radical alkyle linéaire ou ramifié comptant de 3 à 16 atomes de carbone (et de préférence de 4 à 16 atomes de carbone et plus préférentiellement encore de 6 à 12 atomes de carbone) ou cycloalkylalkyle,
ou encore R¹ représente un radical aralkyle éventuellement substitué de 1 à 3 fois sur le groupe aryle par des substituants choisis indépendamment parmi un atome halogène et un radical alkyle ou alkoxy ;
R² représente un radical phényle éventuellement substitué de 1 à 3 fois par des substituants choisis indépendamment parmi un atome halogène et un radical alkyle ou alkoxy,
ou encore R² représente un radical biphényle éventuellement substitué de 1 à 4 fois par un ou des substituants choisis indépendamment parmi un atome halogène et un radical alkyle ou alkoxy ;
R³ représente un atome d'hydrogène ou un radical alkyle ;
et les sels pharmaceutiquement acceptables de ces derniers ;
peuvent être utilisés pour préparer un médicament destiné à moduler les canaux sodiques.

Par alkyle, lorsqu'il n'est pas donné plus de précision, on entend un radical alkyle linéaire ou ramifié comptant de 1 à 12 atomes de carbone, et de préférence de 1 à 6 atomes de carbone. Par cycloalkyle, lorsqu'il n'est pas donné plus de précision, on entend un système monocyclique carboné comptant de 3 à 7 atomes de carbone. Par aryle, lorsqu'il n'est pas donné plus de précision, on entend un radical aryle carbocyclique. Par aryle carbocyclique, on entend un système carbocyclique comprenant au moins un cycle aromatique (et, en particulier, le radical phényle, qui peut être noté de façon abrégée Ph, ou le radical naphtyle).

Par radicaux alkoxy, aralkyle et cycloalkylalkyle, on entend respectivement les radicaux alkoxy, aralkyle et cycloalkylalkyle dont le radical alkyle a la signification indiquée précédemment. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par atome halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm*. (1986), **33**, 201-217.

De préférence, les composés de formule générale **(I)** seront tels qu'ils possèderont au moins l'une des caractéristiques suivantes :
◆ R¹ représentant un radical alkyle linéaire ou ramifié comptant de 3 à 16 atomes de carbone ou cycloalkylalkyle, ou encore R¹ représentant un radical aralkyle éventuellement substitué de 1 à 3 fois sur le groupe aryle par des substituants choisis indépendamment parmi des radicaux alkyle ;
◆ R² représentant un radical phényle éventuellement substitué de 1 à 3 fois par des substituants choisis indépendamment parmi un atome halogène et un radical alkyle, ou encore R² représentant un radical biphényle éventuellement substitué de 1 à 4 fois par un ou des substituants choisis indépendamment parmi un atome halogène et un radical alkyle ;
◆ R³ représentant un atome d'hydrogène.

Plus préférentiellement, les composés de formule générale **(I)** seront tels qu'ils possèderont au moins l'une des caractéristiques suivantes :
◆ R¹ représentant un radical alkyle linéaire ou ramifié comptant de 4 à 16 atomes de carbone ou un radical cycloalkylalkyle dans lequel le radical alkyle compte de 1 à 4 atomes de carbone, ou encore R¹ représentant un radical aralkyle dans lequel le radical alkyle compte de 1 à 4 atomes de carbone, ledit radical aralkyle éventuellement substitué de 1 à 3 fois sur le groupe aryle par des substituants choisis indépendamment parmi des radicaux alkyle ;
◆ R² représentant un radical phényle éventuellement substitué de 1 à 3 fois par des substituants choisis indépendamment parmi un atome halogène et un radical alkyle,
ou encore R² représentant un radical biphényle éventuellement substitué de 1 à 4 fois par un ou des atomes halogènes ;
◆ R³ représentant un atome d'hydrogène.

Encore plus préférentiellement, les composés de formule générale **(I)** seront tels qu'ils possèderont au moins l'une des caractéristiques suivantes :
◆ R' représentant un radical alkyle linéaire ou ramifié comptant de 6 à 16 atomes de carbone ou un radical cycloalkylalkyle dans lequel le radical alkyle compte de 1 à 4 atomes de carbone, ou encore R¹ représentant un radical aralkyle dans lequel le radical alkyle compte de 1 à 4 atomes de carbone, ledit radical aralkyle éventuellement substitué de 1 à 3 fois sur le groupe aryle par des substituants choisis indépendamment parmi des radicaux alkyle ;
◆ R² représentant un radical phényle éventuellement substitué de 1 à 3 fois par des substituants choisis indépendamment parmi un atome halogène et un radical alkyle,
ou encore R² représentant un radical biphényle éventuellement substitué par un atome halogène ;
◆ R³ représentant un atome d'hydrogène.

De façon particulièrement préférée, les composés de formule générale **(I)** seront tels que R¹ représente un radical alkyle ramifié comptant de 6 à 16 atomes de carbone, plus particulièrement de 7 à 16 atomes de carbone et encore plus spécialement de 8 à 16 atomes de carbone (et notamment de 8 à 12 atomes de carbone). En particulier, on préférera les cas où R¹ représentera un radical -CHR⁴R⁵ dans lequel R⁴ représentera un radical -(CH₂)ₚ-CH₃ et R⁵ représentera un radical -(CH₂)ₚ-CH₃, p et q étant des entiers tels que leur somme p + q soit supérieure ou égale à 7 et inférieure ou égale à 16, et de préférence de supérieure ou égale à 8 ou 10 et inférieure ou égale à 12 ou 16.

Également de façon particulièrement préférée, les composés de formule générale **(I)** seront tels que R¹ représente un radical cycloalkylalkyle, dans lequel le radical alkyle compte de préférence de 1 à 4 atomes de carbone (le radical cycloalkyle étant de préférence un radical cyclohexyle).

Toujours de façon particulièrement préférée, les composés de formule générale **(I)** seront tels que R² représente un radical biphényle éventuellement substitué par un atome halogène ou un radical alkyle (et de préférence un radical biphényle substitué par un atome halogène ou un radical alkyle, en particulier un radical biphényle substitué par un atome halogène).

Selon l'invention, les composés de formule générale **(I)** peuvent en particulier être utilisés pour préparer un médicament destiné au traitement ou à la prévention de la douleur, au traitement ou à la prévention de l'épilepsie, au traitement des troubles du rythme cardiaque, au traitement de troubles liés à la neurodégénération, au traitement de la dépression et des troubles bipolaires, au traitement du syndrome du colon irritable ou au traitement des rétinopathies diabétiques. De préférence, le médicament préparé sera destiné au traitement ou à la prévention de la douleur ou de l'épilepsie (et plus préférentiellement au traitement ou à la prévention de la douleur).

En particulier, l'invention concerne les utilisations susmentionnées employant les composés de formule générale **(I)** suivants (décrits dans les exemples) :
- 4-(1,1'-biphényl-4-yl)-2-(4-isobutylbenzyl)-1*H*-imidazole ;
- 2-hexyl-4-(4-isobutylphényl)-1H-imidazole ;
- 4-phényl-2-(3-phénylpropyl)-1H-imidazole ;
- 2-(1-pentylhexyl)-4-phényl-1H-imidazole ;
- 4-(4-fluorophényl)-2-(1-pentylhexyl)-1H-imidazole ;
- 2-(cylohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole;
- 4-(1,1'-biphényl-4-yl)-2-(2-cyclohexyléthyl)-1H-imidazole ;
- 4-(1,1'-biphényl-4-yl)-2-hexyl-1H-imidazole ;
- 4-(1,1'-biphényl-4-yl)-2-[1-(4-isobutylphényl)éthyl]-1H-imidazole ;
- 4-(4-fluorophényl)-2-[1-(4-isobutylphényl)éthyl]-1H-imidazole ;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ainsi que les sels pharmaceutiquement acceptables de ces derniers.

Pour cette utilisation, les composés suivants seront préférés :
- 4-(1,1'-biphényl-4-yl)-2-(4-isobutylbenzyl)-1*H*-imidazole ;
- 2-hexyl-4-(4-isobutylphényl)-1H-imidazole ;
- 2-(1-pentylhexyl)-4-phényl-1H-imidazole ;
- 4-(4-fluorophényl)-2-(1-pentylhexyl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-hexyl-1H-imidazole ;
- 4-(1,1'-biphényl-4-yl)-2-[1-(4-isobutylphényl)éthyl]-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ainsi que les sels pharmaceutiquement acceptables de ces derniers.

L'invention concerne également, à titre de médicaments, les composés de formule générale **(II)** 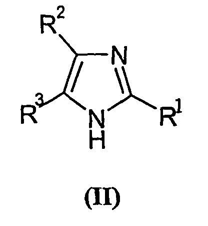 dans laquelle
R¹ représente un radical alkyle linéaire ou ramifié comptant de 3 à 16 atomes de carbone (et de préférence de 4 à 16 atomes de carbone et plus préférentiellement encore de 6 à 12 atomes de carbone) ou cycloalkylalkyle,
ou encore R¹ représente un radical aralkyle éventuellement substitué de 1 à 3 fois sur le groupe aryle par des substituants choisis indépendamment parmi un atome halogène et un radical alkyle ou alkoxy ;
R² représente un radical phényle éventuellement substitué de 1 à 3 fois par des substituants choisis indépendamment parmi un atome halogène et un radical alkyle ou alkoxy,
ou encore R² représente un radical biphényle éventuellement substitué de 1 à 4 fois par un ou des substituants choisis indépendamment parmi un atome halogène et un radical alkyle ou alkoxy ;
R³ représente un atome d'hydrogène ou un radical alkyle ;
étant entendu toutefois que :
- lorsque R² représente un radical phényle éventuellement substitué de 1 à 3 fois par des substituants choisis indépendamment parmi un atome halogène et un radical alkyle ou alkoxy, alors R¹ ne représente ni un radical alkyle linéaire ou ramifié comptant de 1 à 6 atomes de carbone ni un radical 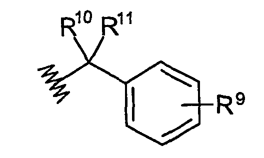
dans lequel R⁹ représente de un à trois substituants choisis indépendamment parmi un atome d'hydrogène, un atome halogène et un radical alkyle ou alkoxy linéaire ou ramifié comptant de 1 à 6 atomes de carbone, et R¹⁰ et R¹¹ étant choisis indépendamment parmi un atome d'hydrogène et un radical alkyle linéaire ou ramifié comptant de 1 à 6 atomes de carbone ;
- lorsque R² représente un radical biphényle non substitué ou lorsque R² représente un radical phényle éventuellement substitué de 1 à 3 fois par des substituants choisis indépendamment parmi un atome halogène et l'un des radicaux alkyle ou alkoxy linéaires ou ramifiés comptant de 1 à 4 atomes de carbone, alors R¹ ne représente ni un radical -(CH₂)ₙ-Z dans lequel n est un entier de 0 à 3 et Z représente un radical alkyle linéaire ou ramifié comptant de 1 à 4 atomes de carbone ou un radical phényle ou naphtyle éventuellement substitué par 1 à 3 substituants choisis parmi le groupe constitué par un atome halogène et les radicaux alkyle ou alkoxy linéaires ou ramifiés comptant de 1 à 4 atomes de carbone ;
ainsi que les sels pharmaceutiquement acceptables de ces derniers.

En particulier, l'invention concerne, à titre de médicaments, les composés de formule générale **(II)** suivants :
- 2-(1-pentylhexyl)-4-phényl-1H-imidazole ;
- 4-(4-fluorophényl)-2-(1-pentylhexyl)-1H-imidazole ;
- 2-(cyclohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-[1-(4-isobutylphényl)éthyl]-1H-imidazole;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ou leurs sels pharmaceutiquement acceptables.

Les préférences énoncées pour les composés de formule générale **(I)** s'appliquent par ailleurs *mutatis mutandis* aux composés de formule générale **(II).**

L'invention concerne également, à titre de médicaments, les composés de formule générale **(I)** ou leurs sels pharmaceutiquement acceptables choisis parmi les composés suivants :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et leurs sels pharmaceutiquement acceptables.

L'invention par ailleurs également concerne les composés de formule générale **(III)** 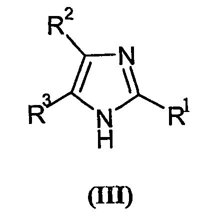 dans laquelle
R¹ représente un radical alkyle linéaire ou ramifié comptant de 3 à 16 atomes de carbone (et de préférence de 4 à 16 atomes de carbone et plus préférentiellement encore de 6 à 12 atomes de carbone) ou cycloalkylalkyle,
ou encore R¹ représente un radical aralkyle éventuellement substitué de 1 à 3 fois sur le groupe aryle par des substituants choisis indépendamment parmi un atome halogène et un radical alkyle ou alkoxy ;
R² représente un radical phényle éventuellement substitué de 1 à 3 fois par des substituants choisis indépendamment parmi un atome halogène et un radical alkyle ou alkoxy,
ou encore R² représente un radical biphényle éventuellement substitué de 1 à 4 fois par un ou des substituants choisis indépendamment parmi un atome halogène et un radical alkyle ou alkoxy ;
R³ représente un atome d'hydrogène ou un radical alkyle ;
étant entendu toutefois que :
- lorsque R² représente un radical biphényle non substitué ou lorsque R² représente un radical phényle éventuellement substitué de 1 à 3 fois par des substituants choisis indépendamment parmi un atome halogène et l'un des radicaux alkyle ou alkoxy linéaires ou ramifiés comptant de 1 à 4 atomes de carbone, alors R¹ ne représente ni un radical -(CH₂)ₙ-Z dans lequel n est un entier de 0 à 3 et Z représente un radical alkyle linéaire ou ramifié comptant de 1 à 4 atomes de carbone ou un radical phényle ou naphtyle éventuellement substitué par 1 à 3 substituants choisis parmi le groupe constitué par un atome halogène et les radicaux alkyle ou alkoxy linéaires ou ramifiés comptant de 1 à 4 atomes de carbone ; et
- lorsque R² représente un radical phényle éventuellement substitué de 1 à 3 fois par des substituants choisis indépendamment parmi un atome halogène et un radical alkyle ou alkoxy, alors R¹ ne représente ni un radical alkyle linéaire ou ramifié comptant de 1 à 6 atomes de carbone ni un radical 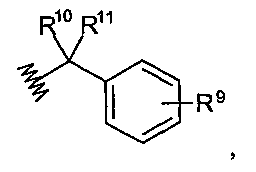 dans lequel R⁹ représente de un à trois substituants choisis indépendamment parmi un atome d'hydrogène, un atome halogène et un radical alkyle ou alkoxy linéaire ou ramifié comptant de 1 à 6 atomes de carbone, et R¹⁰ et R¹¹ étant choisis indépendamment parmi un atome d'hydrogène et un radical alkyle linéaire ou ramifié comptant de 1 à 6 atomes de carbone ;
ainsi que les sels de ces derniers.

En particulier, l'invention concerne, à titre de produits industriels nouveaux, les composés de formule générale **(III)** suivants :
- 2-(1-pentylhexyl)-4-phényl-1H-imidazole ;
- 4-(4-fluorophényl)-2-(1-pentylhexyl)-1H-imidazole ;
- 2-(cyclohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-[1-(4-isobutylphényl)éthyl]-1H-imidazole ;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ou leurs sels.

Les préférences énoncées pour les composés de formule générale **(I)** s'appliquent par ailleurs *mutatis mutandis* aux composés de formule générale **(III)**, excepté toutefois pour les sels qui ne sont pas nécessairement des sels pharmaceutiquement acceptables.

L'invention concerne également, à titre de produits industriels nouveaux, les composés de formule générale **(I)** ou leurs sels choisis parmi :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et leurs sels.

L'invention concerne de plus les compositions pharmaceutiques comprenant, à titre de principe actif, au moins un composé de formule générale **(II)** ou **(III)** telle que définie précédemment ou un sel pharmaceutiquement acceptable d'un tel composé. Elle a aussi pour objet les compositions pharmaceutiques comprenant, à titre de principe actif, au moins un composé décrit dans les exemples 1 à 18 (parfois sous forme de sels) ou un sel pharmaceutiquement acceptable d'un tel composé.

L'invention concerne aussi en particulier les compositions pharmaceutiques contenant, à titre de principe actif, un composé de formule générale **(I)** ou un de ses sels pharmaceutiquement acceptables choisis parmi :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et leurs sels pharmaceutiquement acceptables.

L'invention concerne encore l'utilisation d'un composé de formule générale **(II)** ou **(III)** telle que définie précédemment ou d'un sel pharmaceutiquement acceptable d'un tel composé pour préparer un médicament destiné à moduler les canaux sodiques.

En ce qui concerne les médicaments, les compositions pharmaceutiques et les utilisations précités, les préférences décrites pour les composés de formule générale **(I)** s'appliquent *mutatis mutandis* aux composés de formules générales **(II)** et **(III).**

Les compositions pharmaceutiques contenant un composé de l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A titre indicatif, la dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-après.

### PREPARATION DES COMPOSES DE L'INVENTION :

Les composés de formule générale **(I)** peuvent être préparés selon les procédures analogues à celles décrites dans la demande WO 01/26656 et/ou selon les procédures exposées ci-après. L'ensemble des composés de formule générale **(II)** ou **(III)** peut bien sûr être préparé de la même façon que pour les composés de formule générale **(I).**

Selon l'invention, les composés de formule générale **(I)** peuvent être obtenus par une voie de synthèse résumée dans le schéma 1 ci-après.

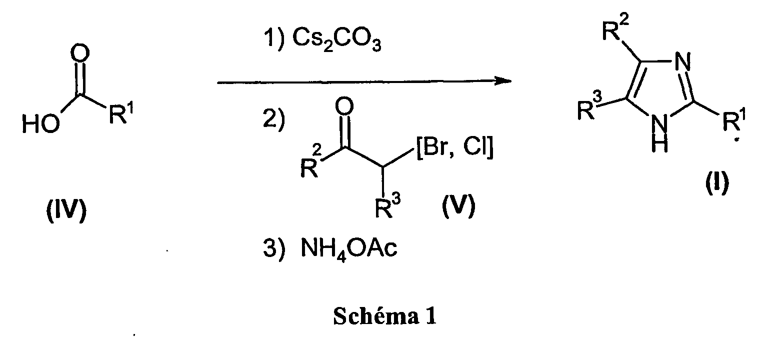

Selon cette méthode de préparation, les acides de formule générale **(IV)** peuvent être cyclisés sous forme de dérivés d'imidazoles de formule générale **(I),** schéma 1, par ajout de carbonate de césium suivi d'une condensation avec une α-halogénocétone de formule générale (V) suivie de l'addition d'un large excès d'acétate d'ammonium (par exemple 15 ou 20 équivalents par équivalent d'acide de formule générale **(IV)**). Cette réaction s'effectue de préférence dans un mélange de xylènes et en chauffant (on peut aussi, le cas échéant, éliminer simultanément l'eau formée au cours de la réaction).

### Préparation de certains acides de formule générale (IV)

Les acides de formule générale **(IV)**_{**1**} 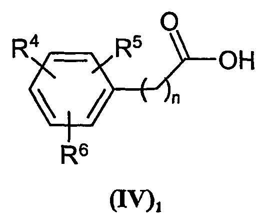 dans laquelle R⁴, R⁵ et R⁶ représentent indépendamment des atomes d'hydrogène ou un radical alkyle et n représente de préférence un entier de 1 à 6 peuvent être préparés par une réaction de Willgerodt-Kindler comme illustré par le schéma 2 ci-dessous.

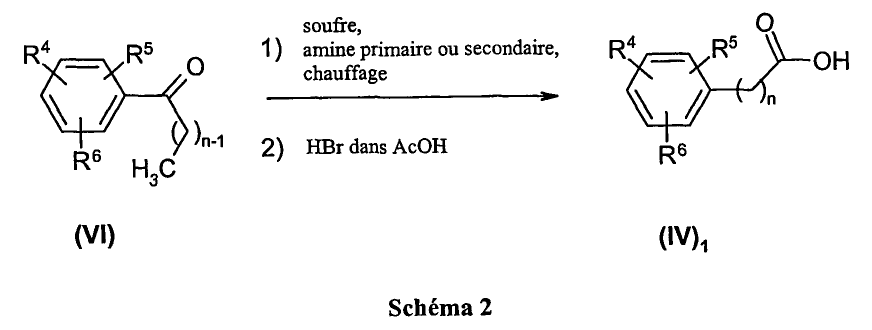

Les arylcétones de formule générale **(VI)** dans lesquelles R⁴, R⁵ et R⁶ ont la même signification que celle indiquée pour la formule générale **(IV)**_{**1**} sont traitées avec du soufre en présence d'un excès d'amine primaire ou secondaire (par exemple la morpholine) en chauffant. Après refroidissement à température ambiante et concentration à sec, une solution d'acide bromhydrique dans de l'acide acétique est ajoutée et l'on obtient l'acide désiré.

### Préparation des α-halogénocétones de formule générale (V)

Les α-halogénocétones de formule générale **(V),** quand elles ne sont pas commerciales, peuvent être préparées à partir des cétones correspondantes par des méthodes d'halogénation connues de l'homme du métier. Par exemple, dans le cas d'une α-bromocétone, on pourra utiliser une réaction avec un agent de bromation tel que CuBr₂ (*J*. *Org. Chem*. (1964), **29**, 3459), du brome (*J. Het. Chem.* (1988), **25**, 337), du N-bromosuccinimide (*J. Amer. Chem. Soc.* (1980), **102**, 2838) en présence d'acide acétique dans un solvant comme l'acétate d'éthyle ou le dichlorométhane, HBr ou Br₂ dans de l'éther, de l'éthanol ou de l'acide acétique *(Biorg. Med. Chem. Lett*. (1996), **6**(3), 253-258 ; *J*. *Med. Chem.* (1988), **31**(10), 1910-1918 ; *J. Am. Chem. Soc.* (1999), **121,** 24) ou encore une résine de bromation telle que la résine PVPHP (Poly(VinylPyridinium Hydrobromide Perbromide) ou poly(perbromure d'hydrobromure de vinylpyridinium)) dans un solvant tel que le méthanol, le dichlorométhane ou le toluène (*J. Macromol. Sci. Chem*. (1977), **A11**, (3) 507-514).

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus.

### EXEMPLES

### Exemple 1 : 4-(1,1'-biphényl-4-yl)-2-(4-isobutylbenzyl)-1H-imidazole :

### 1.1) Acide (4-isobutylphényl)acétique :

Un mélange contenant de la *para*-isobutylacétophénone (7 g ; 40 mmol), 10 ml de morpholine et du soufre (2,45 g ; 78 mmol) est porté à reflux pendant 18 heures. On concentre ensuite à sec et rajoute 50 ml d'une solution à 33% d'acide bromhydrique dans de l'acide acétique. Le mélange résultant est chauffé à reflux pendant 3 h puis on laisse la température revenir à 23 °C. Le milieu réactionnel, auquel sont ajoutées de petites portions de toluène, est à nouveau concentré à sec. Le résidu est repris dans de l'acétone et les sels filtrés sur fritté. Du noir animal est ajouté et la suspension chauffée à reflux pendant deux heures. On filtre sur célite et concentre à sec le filtrat avant de procéder à une distillation sous vide (P# 0,7 torr ; T # 125-130 °C). On obtient un solide de couleur jaune à température ambiante (rendement de 33%). Point de fusion : 78-80 °C.

### 1.2) 4-(1,1'-biphényl-4-yl)-2-(4-isobutylbenzyl)-1H-imidazole :

Un mélange contenant l'intermédiaire 1.1 préparé précédemment (1,2 g; 6,76 mmol) avec du carbonate de césium (1,1 g ; 3,38 mmol) dans 30 ml de méthanol est agité à température ambiante pendant une heure. Le solvant est évaporé puis le résidu repris dans 30 ml de diméthylformamide. On ajoute ensuite de la 2-bromo-4'-phénylacétophénone (1,85 g ; 6,76 mmol) et laisse le mélange agité durant 18 h. On concentre à sec et reprend le résidu dans 80 ml de xylène. Après filtration sur sur fritté, on rajoute au filtrat de l'acétate d'ammonium (10,4 g ; 135 mmol). On chauffe le mélange obtenu à reflux pendant 2 h en éliminant l'eau avec un dispositif Dean Stark.

Une fois le mélange réactionnel revenu à température ambiante, celui-ci est versé dans de l'eau glacée. On neutralise l'acétate d'ammonium avec une solution saturée en NaHCO₃ avant de laisser décanter les phases et d'extraire à l'aide d'acétate d'éthyle. La phase organique est ensuite avec une solution saturée en chlorure de sodium, séchée sur sulfate de magnésium et concentrée à sec. Après purification sur une colonne de silice (éluant : acétate d'éthyle-heptane : 2-8 à 6-4), on obtient une poudre de couleur jaune (rendement de 52%). Point de fusion : 160-161 °C. MH+ = 367,3.

### Exemple 2 : 2-hexyl-4-(4-isobutylphényl)-1H-imidazole :

### 2.1) 2-bromo-1-(4-isobutylphényl)éthanone :

Du brome (1,25 ml; 25 mmol) est ajouté goutte à goutte à une solution de 4'-isobutylacétophénone (3,52 g; 20 mmol) dans 60 ml d'éthanol refroidie aux environs de 5 °C. Après 30 minutes d'agitation du mélange réactionnel à cette température, on poursuit l'agitation durant une heure à température ambiante. Le milieu réactionnel, auquel sont ajoutées de petites portions de toluène, est concentré à sec. Après purification sur une colonne de silice (éluant : acétate d'éthyle-heptane : 5-95), on obtient une huile incolore avec un rendement de 57%.
RMN ¹H (400MHz) : 0,85 (d, 6H) ; 1,84-1,92 (s, 1H) ; 2,53 (d, 2H) ; 4,89 ( s, 2H) ; 7,33 (d, 2H) ; 7,93 (d, 2H).

### 2.2) 2-hexyl-4-(4-isobutylphényl)-1H-imidazole :

Ce composé est obtenu selon un mode opératoire analogue à celui décrit pour l'étape 1.2 de l'exemple 1. On obtient une poudre de couleur blanche avec un rendement de 38%. Point de fusion : 89-90 °C. MH+ = 285,2.

*Les composés des exemples 3 à 18 sont préparés à partir de produits commerciaux selon des modes opératoires analogues à ceux des exemples 1 et 2, éventuellement complétés par les modes opératoires généraux décrits dans la partie « Préparation des composés de l'invention » (notamment pour ce qui concerne la bromation en position α des cétones).*

### Exemple 3 : 4-phényl-2-(3-phénylpropyl)-1H-imidazole :

Poudre blanche. Point de fusion : 106-108 °C.

### Exemple 4 : 2-(1-pentylhexyl)-4-phényl-1H-imidazole :

Poudre blanche. Point de fusion : 111,2 °C.

### Exemple 5 : 4-(4-fluorophényl)-2-(1-pentylhexyl)-1H-imidazole :

Poudre blanche. Point de fusion : 134,6 °C.

### Exemple 6 : 2-(cyclohexylméthyl)-4-(4-fluorophényl)-1H-imidazole :

Mousse jaune clair. Point de fusion : 68-70 °C.

### Exemple 7 : 4-(1,1'-biphényl-4-yl)-2-(1-pentylhexyl)-1H-imidazole :

Poudre crème clair. Point de fusion : 173,6 °C.

### Exemple 8 : 4-(4-tert-butylphényl)-2-(1-propylbutyl)-1H-imidazole :

Poudre blanche. Point de fusion : 200-202 °C.

### Exemple 9 : 4-(4-fluorophényl)-2-(1-propylbutyl)-1H-imidazole :

Poudre crème clair. Point de fusion : 217,5 °C.

### Exemple 10 : 4-(4'-bromo-1,1'-biphényl-4-yl)-2-(1-propylbutyl)-1H-imidazole :

Poudre crème clair. Point de fusion: 182,8 °C.

### Exemple 11 : 4-(1,1'-biphényl-4-yl)-2-(2-cyclohexyléthyl)-1H-imidazole :

Poudre crème clair. Point de fusion : 104,9 °C.

### Exemple 12 : 4-(1,1'-biphényl-4-yl)-2-hexyl-1H-imidazole :

Poudre crème clair. Point de fusion: 104,8 °C.

### Exemple 13 : 4-(1,1'-biphényl-4-yl)-2-[1-(4-isobutylphényl)éthyl]-1H-imidazole :

Poudre jaune clair. Point de fusion : 172,7 °C.

### Exemple 14 : 4-(4-fluorophényl)-2-[1-(4-isobutylphényl)éthyl]-1H-imidazole :

Poudre crème clair. Point de fusion : 116 °C.

### Exemple 15 : 4-(1,1'-biphényl-4-yl)-2-(1-phényléthyl)-1H-imidazole :

Poudre jaune clair. Point de fusion : 165-166 °C.

### Exemple 16 : 4-(1,1'-biphényl-4-yl)-2-(1-propylbutyl)-1H-imidazole :

Poudre jaune clair. Point de fusion : 197-198 °C.

### Exemple 17 : 4-(1,1'-biphényl-4-yl)-2-(cyclohexylméthyl)-1H-imidazole :

Poudre crème clair. Point de fusion : 104-105 °C.

### Exemple 18 : 4-(1,1'-biphényl-4-yl)-2-(3-cyclohexylpropyl)-1H-imidazole :

Poudre crème clair. Point de fusion : 118-119 °C.

### Etude pharmacologique des produits de l'invention

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (*J. Neurosci.* (1986), **6**, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M, K₂HPO₄ 5 mM, pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM, KCl 5,4 mM, MgSO₄ 0,8 mM, glucose 5,5 mM, chlorure de choline 130 mM pH 7,4) puis aliquoté et conservé à - 80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de vératridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM, CaCl₂ 1,8 mM, MgSO₄ 0,8 mM, chlorure de choline 130 mM, pH 7,4). Après avoir ajouté 20 µl de Microscint 0 ®, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1 à 8 et 10 à 18 décrits ci-dessus présentent tous une CI₅₀ inférieure ou égale à 1 µM.

## Revendications

1. Utilisation d'un composé de formule générale **(I)** 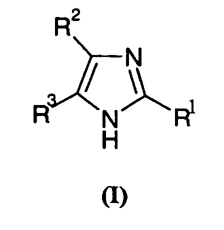 dans laquelle
R¹ représente un radical alkyle linéaire ou ramifié comptant de 3 à 16 atomes de carbone ou un radical cycloalkylalkyle dont le radical cycloalkyle compte de 3 à 7 atomes de carbone et le radical alkyle est linéaire ou ramifié et compte de 1 à 6 atomes de carbone,
ou encore R¹ représente un radical aralkyle éventuellement substitué de 1 à 3 fois sur le groupe aryle par des substituants choisis indépendamment parmi un atome halogène et un radical alkyle ou alkoxy linéaire ou ramifié et comptant de 1 à 6 atomes de carbone ;
R² représente un radical phényle éventuellement substitué de 1 à 3 fois par des substituants choisis indépendamment parmi un atome halogène et un radical alkyle ou alkoxy linéaire ou ramifié et comptant de 1 à 6 atomes de carbone,
ou encore R² représente un radical biphényle éventuellement substitué de 1 à 4 fois par un ou des substituants choisis indépendamment parmi un atome halogène et un radical alkyle ou alkoxy linéaire ou ramifié et comptant de 1 à 6 atomes de carbone ;
R³ représente un atome d'hydrogène ou un radical alkyle linéaire ou ramifié et comptant de 1 à 6 atomes de carbone ;
ou d'un sel pharmaceutiquement acceptable d'un composé de formule générale **(I)**
pour préparer un médicament destiné au traitement ou à la prévention de la douleur, au traitement de l'épilepsie, au traitement des troubles du rythme cardiaque, au traitement des accidents cérébraux vasculaires, au traitement des traumatismes cérébraux, au traitement des maladies neurodégénératives, au traitement de la dépression et des troubles bipolaires ou au traitement du syndrome du colon irritable.

2. Utilisation selon la revendication 1, **caractérisée en ce que** le médicament préparé est destiné au traitement ou à la prévention de la douleur, au traitement de l'épilepsie, au traitement des troubles du rythme cardiaque, au traitement de la dépression et des troubles bipolaires ou au traitement du syndrome du colon irritable.

3. Utilisation selon la revendication 2, **caractérisée en ce que** le médicament préparé est destiné au traitement ou à la prévention de la douleur ou de l'épilepsie.

4. Utilisation selon l'une des revendications 1 à 3, **caractérisée en ce que** le composé utilisé est l'un des composés suivants :
- 4-(1,1'-biphényl-4-yl)-2-(4-isobutylbenzyl)-1*H*-imidazole ;
- 2-hexyl-4-(4-isobutylphényl)-1H-imidazole ;
- 4-phényl-2-(3-phénylpropyl)-1H-imidazole ;
- 2-(1-pentylhexyl)-4-phényl-1H-imidazole ;
- 4-(4-fluorophényl)-2-(1-pentylhexyl)-1H-imidazole ;
- 2-(cyclohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-hexyl-1H-imidazole ;
- 4-(1,1'-biphényl-4-yl)-2-[1-(4-isobutylphényl)éthyl]-1H-imidazole ;
- 4-(4-fluorophényl)-2-[1-(4-isobutylphényl)éthyl]-1H-imidazole ;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ou un sel pharmaceutiquement acceptable d'un de ces derniers.

5. Utilisation selon la revendication 4, **caractérisée en ce que** le composé utilisé est l'un des composés suivants :
- 4-(1,1'-biphényl-4-yl)-2-(4-isobutylbenzyl)-1*H*-imidazole ;
- 2-hexyl-4-(4-isobutylphényl)-1H-imidazole ;
- 2-(1-pentylhexyl)-4-phényl-1H-imidazole ;
- 4-(4-fluorophényl)-2-(1-pentylhexyl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-hexyl-1H-imidazole ;
- 4-(1,1'-biphényl-4-yl)-2-[1-(4-isobutylphényl)éthyl]-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ou un sel pharmaceutiquement acceptable d'un de ces derniers.

6. A titre de médicament, un composé de formule générale **(II)** 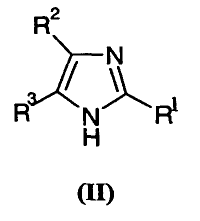 dans laquelle
R¹ représente un radical alkyle linéaire ou ramifié comptant de 3 à 16 atomes de carbone ou un radical cycloalkylalkyle dont le radical cycloalkyle compte de 3 à 7 atomes de carbone et le radical alkyle est linéaire ou ramifié et compte de 1 à 6 atomes de carbone,
ou encore R¹ représente un radical aralkyle éventuellement substitué de 1 à 3 fois sur le groupe aryle par des substituants choisis indépendamment parmi un atome halogène et un radical alkyle ou alkoxy linéaire ou ramifié et comptant de 1 à 6 atomes de carbone ;
R² représente un radical phényle éventuellement substitué de 1 à 3 fois par des substituants choisis indépendamment parmi un atome halogène et un radical alkyle ou alkoxy linéaire ou ramifié et comptant de 1 à 6 atomes de carbone,
ou encore R² représente un radical biphényle éventuellement substitué de 1 à 4 fois par un ou des substituants choisis indépendamment parmi un atome halogène et un radical alkyle ou alkoxy linéaire ou ramifié et comptant de 1 à 6 atomes de carbone ;
R³ représente un atome d'hydrogène ou un radical alkyle linéaire ou ramifié et comptant de 1 à 6 atomes de carbone ;
étant entendu toutefois que :
- lorsque R² représente un radical phényle éventuellement substitué de 1 à 3 fois par des substituants choisis indépendamment parmi un atome halogène et un radical alkyle ou alkoxy, alors R¹ ne représente ni un radical alkyle linéaire ou ramifié comptant de 1 à 6 atomes de carbone ni un radical 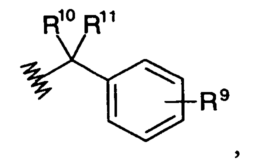 dans lequel R⁹ représente de un à trois substituants choisis indépendamment parmi un atome d'hydrogène, un atome halogène et un radical alkyle ou alkoxy linéaire ou ramifié comptant de 1 à 6 atomes de carbone, et R¹⁰ et R¹¹ étant choisis indépendamment parmi un atome d'hydrogène et un radical alkyle linéaire ou ramifié comptant de 1 à 6 atomes de carbone ;
- lorsque R² représente un radical biphényle non substitué ou lorsque R² représente un radical phényle éventuellement substitué de 1 à 3 fois par des substituants choisis indépendamment parmi un atome halogène et l'un des radicaux alkyle ou alkoxy linéaires ou ramifiés comptant de 1 à 4 atomes de carbone, alors R¹ ne représente ni un radical -(CH₂)ₙ-Z dans lequel n est un entier de 0 à 3 et Z représente un radical alkyle linéaire ou ramifié comptant de 1 à 4 atomes de carbone ou un radical phényle ou naphtyle éventuellement substitué par 1 à 3 substituants choisis parmi le groupe constitué par un atome halogène et les radicaux alkyle ou alkoxy linéaires ou ramifiés comptant de 1 à 4 atomes de carbone ;
ou un sel pharmaceutiquement acceptable d'un composé de formule générale **(II).**

7. Médicament selon la revendication 6, **caractérisé en ce qu'**il s'agit de l'un des composés suivants :
- 2-(1-pentylhexyl)-4-phényl-1H-imidazole ;
- 4-(4-fluorophényl)-2-(1-pentylhexyl)-1H-imidazole ;
- 2-(cyclohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-[1-(4-isobutylphényl)éthyl]-1H-imidazole ;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
ou un sel pharmaceutiquement acceptable d'un de ces derniers.

8. A titre de médicament, un composé de formule générale **(I)** telle que définie dans la revendication 1, ou son sel pharmaceutiquement acceptable, **caractérisé en ce qu'**il est choisi parmi :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et les sels pharmaceutiquement acceptables de ces derniers.

9. Composition pharmaceutique comprenant, à titre de principe actif, un composé de formule générale **(II)** telle que définie dans la revendication 6, ou un sel pharmaceutiquement acceptable d'un tel composé.

10. Composition pharmaceutique comprenant, à titre de principe actif, un composé de formule générale **(I)** telle que définie dans la revendication 1, ou un sel pharmaceutiquement acceptable d'un tel composé **caractérisé en ce qu'**il est choisi parmi :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et les sels pharmaceutiquement acceptables de ces derniers.

11. Composé de formule générale **(III)** 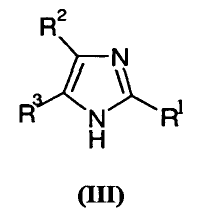 dans laquelle
R¹ représente un radical alkyle linéaire ou ramifié comptant de 3 à 16 atomes de carbone ou un radical cycloalkylalkyle dont le radical cycloalkyle compte de 3 à 7 atomes de carbone et le radical alkyle est linéaire ou ramifié et compte de 1 à 6 atomes de carbone,
ou encore R¹ représente un radical aralkyle éventuellement substitué de 1 à 3 fois sur le groupe aryle par des substituants choisis indépendamment parmi un atome halogène et un radical alkyle ou alkoxy linéaire ou ramifié et comptant de 1 à 6 atomes de carbone ;
R² représente un radical phényle éventuellement substitué de 1 à 3 fois par des substituants choisis indépendamment -parmi un atome halogène et un radical alkyle ou alkoxy linéaire ou ramifié et comptant de 1 à 6 atomes de carbone,
ou encore R² représente un radical biphényle éventuellement substitué de 1 à 4 fois par un ou des substituants choisis indépendamment parmi un atome halogène et un radical alkyle ou alkoxy linéaire ou ramifié et comptant de 1 à 6 atomes de carbone ;
R³ représente un atome d'hydrogène ou un radical alkyle linéaire ou ramifié et comptant de 1 à 6 atomes de carbone ;
étant entendu toutefois que :
- lorsque R² représente un radical phényle éventuellement substitué de 1 à 3 fois par des substituants choisis indépendamment parmi un atome halogène et un radical alkyle ou alkoxy, alors R¹ ne représente ni un radical alkyle linéaire ou ramifié comptant de 1 à 6 atomes de carbone ni un radical 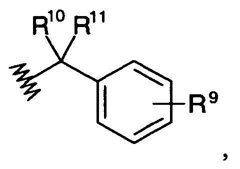 dans lequel R⁹ représente de un à trois substituants choisis indépendamment parmi un atome d'hydrogène, un atome halogène et un radical alkyle ou alkoxy linéaire ou ramifié comptant de 1 à 6 atomes de carbone, et R¹⁰ et R¹¹ étant choisis indépendamment parmi un atome d'hydrogène et un radical alkyle linéaire
ou ramifié comptant de 1 à 6 atomes de carbone ;
- lorsque R² représente un radical biphényle non substitué ou lorsque R² représente un radical phényle éventuellement substitué de 1 à 3 fois par des substituants choisis indépendamment parmi un atome halogène et l'un des radicaux alkyle ou alkoxy linéaires ou ramifiés comptant de 1 à 4 atomes de carbone, alors R¹ ne représente ni un radical -(CH₂)ₙ-Z dans lequel n est un entier de 0 à 3 et Z représente un radical alkyle linéaire ou ramifié comptant de 1 à 4 atomes de carbone ou un radical phényle ou naphtyle éventuellement substitué par 1 à 3 substituants choisis parmi le groupe constitué par un atome halogène et les radicaux alkyle ou alkoxy linéaires ou ramifiés comptant de 1 à 4 atomes de carbone ;
ou un sel d'un de ces composés.

12. Composé selon la revendication 11, **caractérisé en ce qu'**il est choisi parmi les composés suivants :
- 2-(1-pentylhexyl)-4-phényl-1H-imidazole ;
- 4-(4-fluorophényl)-2-(1-pentylhexyl)-1H-imidazole ;
- 2-(cyclohexylméthyl)-4-(4-fluorophényl)-1H-imidazole ;
- 4-(1,1'-biphényl-4-yl)-2-(1-pentylhexyl)-1H-imidazole ;
- 4-(4-tert-butylphényl)-2-(1-propylbutyl)-1H-imidazole ;
- 4-(4-fluorophényl)-2-(1-propylbutyl)-1H-imidazole ;
- 4-(4'-bromo-1,1'-biphényl-4-yl)-2-(1-propylbutyl)-1H-imidazole ;
- 4-(1,1'-biphényl-4-yl)-2-(2-cyclohexyléthyl)-1H-imidazole ;
- 4-(1,1'-biphényl-4-yl)-2-[1-(4-isobutylphényl)éthyl]-1H-imidazole ;
- 4-(1,1'-biphényl-4-yl)-2-(1-phényléthyl)-1H-imidazole ;
- 4-(1,1'-biphényl-4-yl)-2-(1-propylbutyl)-1H-imidazole ;
- 4-(1,1'-biphényl-4-yl)-2-(cyclohexylméthyl)-1H-imidazole ;
- 4-(1,1'-biphényl-4-yl)-2-(3-cyclohexylpropyl)-1H-imidazole ;
et les sels de ces composés.

13. Composé de formule générale **(I)** telle que définie dans la revendication 1, ou sel d'un tel composé, **caractérisé en ce qu'**il est choisi parmi :
- le 4-(1,1'-biphényl-4-yl)-2-(4-isobutylbenzyl)-1*H*-imidazole ;
- le 2-hexyl-4-(4-isobutylphényl)-1H-imidazole ;
- le 4-phényl-2-(3-phénylpropyl)-1H-imidazole ;
- le 4-(1,1'-biphényl-4-yl)-2-hexyl-1H-imidazole ;
- le 4-(4-fluorophényl)-2-[1-(4-isobutylphényl)éthyl]-1H-imidazole ;
et les sels de ces derniers.

## Patentansprüche

1. Verwendung einer Verbindung der allgemeinen Formel (I) 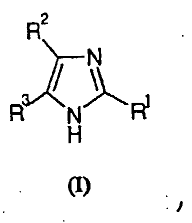 in der
R¹ einen linearen oder verzweigten Alkylrest, der 3 bis 16 Kohlenstoffatome zählt, oder einen Cycloalkylalkylrest darstellt, bei dem der Cycloalkylrest 3 bis 7 Kohlenstoffatome zählt und der Alkylrest linear oder verzweigt ist und 1 bis 6 Kohlenstoffatome zählt,
oder außerdem R¹ einen Aralkylrest darstellt, der gegebenenfalls 1- bis 3-fach an der Arylgruppe substiuiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt;
R² einen Phenylrest darstellt, der gegebenenfalls 1- bis 3-fach substituiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt,
oder außerdem R² einen Biphenylrest darstellt, der gegebenenfalls 1- bis 4-fach substiuiert ist mit einem oder mehreren Substituenten, die unabhängig voneinander ausgewählt sind aus einem Halogenatom und einem linearen oder verzweigten Alkyl- oder Alkoxyrest, der 1 bis 6 Kohlenstoffatome zählt;
R³ ein Wasserstoffatom oder einen linearen oder verzweigten Alkylrest darstellt, der 1 bis 6 Kohlenstoffatome zählt;
oder eines pharmazeutisch annehmbaren Salzes einer Verbindung der allgemeinen Formel (I)
zur Herstellung eines Medikamentes, das zur Behandlung oder Vorbeugung von Schmerz, zur Behandlung von Epilepsie, zur Behandlung von Herzrhythmusstörungen, zur Behandlung von zerebralen Gefäßerkrankungen, zur Behandlung von zerebralen Traumata, zur Behandlung von neurodegenerativen Krankheiten, zur Behandlung von Depression und manischer Depression oder zur Behandlung des Reizdarmsyndroms bestimmt ist.

2. Verwendung nach Anspruch 1, **dadurch gekennzeichnet, dass** das hergestellte Medikament zur Behandlung oder Vorbeugung von Schmerz, zur Behandlung von Epilepsie, zur Behandlung von Herzrythmusstörungen, zur Behandlung von Depression und manischer Depression oder zur Behandlung des Reizdarmsyndroms bestimmt ist.

3. Verwendung nach Anspruch 2, **dadurch gekennzeichnet, dass** das hergestellte Medikament zur Behandlung oder Vorbeugung von Schmerz oder von Epilepsie bestimmt ist.

4. Verwendung nach einem der Ansprüche 1 - 3, **dadurch gekennzeichnet, dass** die verwendete Verbindung eine der folgenden Verbindungen ist:
- 4-(1,1'-Biphenyl-4-yl)-2-(4-isobutylbenzyl)-1H-imidazol;
- 2-Hexyl-4-(4-isobutylphenyl)-1H-imidazol;
- 4-Phenyl-2-(3-phenylpropyl)-1H-imidazol;
- 2-(1-Pentylhexyl1)-4-phenyl-1H-imidazol;
- 4-(4-Fluorphenyl)-2-(1-pentylhexyl)-1H-imidazol;
- 2-(Cyclohexylmethyl)-4-(4-fluorphenyl)-1H-imidazol;
- 4-(1,1'-Biphenyl-4-yl)-2-(1-pentylhexyl)-1H-imidazol;
- 4-(4-tert-Butylphenyl)-2-(1-propylbutyl)-1H-imidazol;
- 4-(4-Fluorphenyl)-2-(1-propylbutyl)-1H-imidazol;
- 4-(4'-Brom-1,1'-biphenyl-4-yl)-2-(1-propylbutyl)-1H-imidazol;
- 4-(1,1'-Biphenyl-4-yl)-2-(2-cyclohexylethyl)-1H-imidazol;
- 4-(1,1'-Biphenyl-4-yl)-2-hexyl-1H-imidazol;
- 4-(1,1'-Biphenyl-4-yl)-2-[1-(4-isobutylphenyl)ethyl]-1H-imidazol;
- 4-(4-Fluorphenyl)-2-[1-(4-isobutylphenyl)ethyl]-1H-imidazol;
- 4-(1,1'-Biphenyl-4-yl)-2-(1-phenylethyl)-1H-imidazol;
- 4-(1,1'-Biphenyl-4-yl)-2-(1-propylbutyl)-1H-imidazol;
- 4-(1,1'-Biphenhyl-4-yl)-2-(cyclohexylmethyl)-1H-imidazol;
- 4-(1,1'-Biphenhyl-4-yl)-2-(3-cyclohexylpropyl)-1H-imidazol;
oder ein pharmazeutisch annehmbares Salz einer dieser Verbindungen.

5. Verwendung nach Anspruch 4, **dadurch gekennzeichnet, dass** die verwendete Verbindung eine der folgenden ist:
- 4-(1,1'-Biphenyl-4-yl)-2-(4-isobutylbenzyl)-1H-imidazol;
- 2-Hexyl-4-(4-isobutylphenyl)-1H-imidazol;
- 2-(1-Pentylhexyl)-4-phenyl-1H-imidazol;
- 4-(4-Fluorphenyl)-2-(1-pentylhexyl)-1H-imidazol;
- 4-(1,1'-Biphenyl-4-yl)-2-(1-pentylhexyl)-1H-imidazol;
- 4-(4-tert-Butylphenyl)-2-(1-propylbutyl)-1H-imidazol;
- 4-(1,1'-Biphenyl-4-yl)-2-(2-cyclohexylethyl)-1H-imidazol;
- 4-(1,1'-Biphenyl-4-yl)-2-hexyl-1H-imidazol;
- 4-(1,1'-Biphenyl-4-yl)-2-[1-(4-isobutylphenyl)ethyl]-1H-imidazol;
- 4-(1,1'-Biphenyl-4-yl)-2-(1-propylbutyl)-1H-imidazol;
- 4-(1,1'-Biphenhyl-4-yl)-2-(cyclohexylmethyl)-1H-imidazol;
- 4-(1,1'-Biphenhyl-4-yl)-2-(3-cyclohexylpropyl)-1H-imidazol;
oder ein pharmazeutisch annehmbares Salz einer dieser Verbindungen.

6. Als Arzneistoff, eine Verbindung der allgemeinen Formel (II) 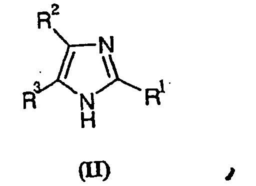 in der
R¹ einen linearen oder verzweigten Alkylrest, der 3 bis 16 Kohlenstoffatome zählt, oder einen Cycloalkylrest darstellt, bei dem der Cycloalkylrest 3 bis 7 Kohlenstoffatome zählt und der Alkylrest linear oder verzweigt ist und 1 bis 6 Kohlenstoffatome zählt;
und außerdem R¹ einen Aralkylrest darstellt, der gegebenenfalls 1- bis 3-fach an der Arylgruppe substituiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt;
R² einen Phenylrest darstellt, der gegebenenfalls 1- bis 3-fach substituiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt,
oder außerdem R² einen Biphenylrest darstellt, der gegebenenfalls 1- bis 4-fach substiuiert ist mit einen oder mehreren Substituenten, die unabhängig voneinander ausgewählt sind aus einem Halogenatom und einem linearen oder verzweigten Alkyl- oder Alkoxyrest, der 1 bis 6 Kohlenstoffatome zählt;
R³ ein Wasserstoffatom oder einen linearen oder verzweigten Alkylrest darstellt, der 1 bis 6 Kohlenstoffatome zählt;
wobei jedoch zu beachten ist, dass:
- wenn R² einen Phenylrest darstellt, der gegebenenfalls 1 bis 3-fach substituiert ist mit Substituenten, die unabhängig voneinander ausgewählt sind aus einem Halogenatom und einem Alkyl- oder Alkoxyrest, dann R¹ weder einen linearen oder verzweigten Alkylrest, der 1 bis 6 Kohlenstoffatome zählt, noch einen Rest 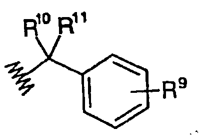 darstellt, bei dem R⁹ von 1 bis 3 Substituenten darstellt, die unabhängig voneinander ausgewählt sind aus einem Wasserstoffatom, einem Halogenatom und einem linearen oder verzweigten Alky- oder Alkoxyrest, der 1 bis 6 Kohlenstoffatome zählt, und bei dem R¹⁰ und R¹¹ unabhängig voneinander ausgewählt sind aus einem Wasserstoffatom und einem linearen oder verzweigten Alkylrest, der 1 bis 6 Kohlenstoffatome zählt;
- wenn R² einen nicht substituierten Biphenylrest darstellt oder wenn R² einen Phenylrest darstellt, der gegebenenfalls 1- bis 3-fach substituiert ist mit Substituenten, die unabhängig voneinander ausgewählt sind aus einem Halogenatom und einem der linearen oder verzweigten Alkyl- oder Alkoxyreste, die 1 bis 4 Kohlenstoffatome zählen, dann R¹ nicht einen -(CH₂)ₙ-Z-Rest darstellt, in dem n eine ganze Zahl von 0 bis 3 ist und Z einen linearen oder verzweigten Alkylrest, der 1 bis 4 Kohlenstoffatome zählt, oder einen Phenyl- oder Naphthylrest darstellt, der gegebenenfalls mit 1 bis 3 Substiuenten substituiert ist, die ausgewählt sind aus der Gruppe bestehend aus einem Halogenatom und einem linearen oder verzweigten Alkyl- oder Alkoxyrest, der 1 bis 4 Kohlenstoffatome zählt;
oder ein pharmazeutisch annehmbares Salz einer Verbindung der allgemeinen Formel (II).

7. Medikament nach Anspruch 6, **dadurch gekennzeichnet, dass** es sich um eine der folgende Verbindungen handelt:
- 2-(1-Pentylhexyl)-4-phenyl-1H-imidazol;
- 4-(4-Fluorphenyl)-2-(1-pentylhexyl)-1H-imidazol;
- 2-(Cyclohexylmethyl)-4-(4-fluorphenyl)-1H-imidazol;
- 4-(1,1'-Biphenyl-4-yl)-2-(1-pentylhexyl)-1H-imidazol;
- 4-(4-tert-Butylphenyl)-2-(1-propylbutyl)-1H-imidazol;
- 4- (4-Fluorphenyl) -2-(1-propylbutyl)-1H-imidazol;
- 4-(4'-Bromo-1,1'-biphenyl-4-yl)-2-(1-propylbutyl)-1H-imidazol;
- 4-(1,1'-Biphenyl-4-yl)-2-(2-cyclohexylethyl)-1H-imidazol;
- 4-(1,1'-Biphenyl-4-yl)-2-[1-(4-isobutylphenyl)etyl]-1H-imidazol;
- 4-(1,1'-Biphenyl-4-yl)-2-(1-phenylethyl)-1H-imidazol;
- 4-(1,1'-Biphenyl-4-yl)-2-(1-propylbutyl)-1H-imidazol;
- 4-(1,1'-Biphenyl-4-yl)-2-(cyclohexylmethyl)-1H-imidazol;
- 4-(1,1'-Biphenyl-4-yl)-2-(3-cyclohexylpropyl)-1H-imidazol;
oder ein pharmazeutisch annehmbares Salz einer dieser Verbindungen.

8. Als Arzneistoff, eine Verbindung der allgemeinen Formel (I), wie sie in Anspruch 1 definiert ist, oder ihre pharmazeutisch annehmbaren Salze, **dadurch gekennzeichnet, dass** sie ausgewählt ist aus:
- 4-(1,1'-Biphenyl-4-yl)-2-(4-isobutylbenzyl)-1H-imidazol;
- 2-Hexyl-4-(4-isobutylphenyl)-1H-imidazol;
- 4-Phenyl-2-(3-phenylpropyl)-1H-imidazol;
- 4-(1,1'-Biphenyl-4-yl)-2-hexyl-1H-imidazol;
- 4-(4-Fluorphenyl)-2-[1-(4-isobutylphenyl)ethyl]-1H-imidazol;
und den pharmazeutisch annehmbaren Salzen dieser Verbindungen.

9. Pharmazeutische Zusammensetzung, die als Wirkstoff eine Verbindung der allgemeinen Formel (II) wie sie in Anspruch 6 definiert ist, oder ein pharmazeutisch annehmbares Salz einer solchen Verbindung umfasst.

10. Pharmazeutische Zusammensetzung, die als Wirkstoff eine Verbindung der allgemeinen Formel (I) wie sie in Anspruch 1 definiert ist, oder ein pharmazeutisch annehmbares Salz einer solchen Verbindung umfasst, **dadurch gekennzeichnet, dass** sie ausgewählt ist aus:
- 4-(1,1'-Biphenyl-4-yl)-2-(4-isobutylbenzyl)-1H-imidazol;
- 2-Hexyl-4-(4-isobutylphenyl)-1H-imidazol;
- 4-Phenyl-2-(3-phenylpropyl)-1H-imidazol;
- 4-(1,1'-Biphenyl-4-yl)-2-hexyl-1H-imidazol;
- 4-(4-Fluorphenyl)-2-[1-(4-isobutylphenyl)ethyl]-1H-imidazol;
und den pharmazeutisch annehmbaren Salzen dieser Verbindungen.

11. Verbindung der allgemeinen Formel (III) 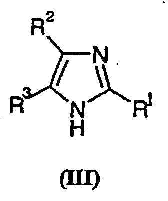 in der
R¹ einen linearen oder verzweigten Alkylrest, der 3 bis 16 Kohlenstoffatome zählt, oder einen Cycloalkylrest darstellt, bei dem der Cycloalkylrest 3 bis 7 Kohlenstoffatome zählt und der Alkylrest linear oder verzweigt ist und 1 bis 6 Kohlenstoffatome zählt,
oder außerdem R¹ einen Aralkylrest darstellt, der gegebenenfalls 1- bis 3-fach an der Arylgruppe substiuiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt;
R² einen Phenylrest darstellt, der gegebenenfalls 1- bis 3-fach substiuiert ist mit Substituenten, die unabhängig voneinander ausgewählt sind aus einem Halogenatom und einem linearen oder verzweigten Alkyl- oder Alkoxyrest, der 1 bis 6 Kohlenstoffatome zählt,
oder außerdem R² einen Biphenylrest darstellt, der gegebenenfalls 1- bis 4-fach substiuiert ist mit einem oder mehreren Substituenten, die unabhängig voneinander ausgewählt sind aus einem Halogenatom und einem linearen oder verzweigten Alkyl- oder Alkoxyrest, der 1 bis 6 Kohlenstoffatome zählt;
R³ ein Wasserstoffatom oder einen linearen oder verzweigten Alkylrest darstellt, der 1 bis 6 Kohlenstoffatome zählt;
wobei jedoch zu beachten ist, dass:
- wenn R² einen Phenylrest darstellt, der gegebenenfalls 1- bis 3-fach substituiert ist mit Substituenten, die unabhängig voneinander ausgewählt sind aus einem Halogenatom und einem Alkyl- oder Alkoxyrest, dann R¹ weder einen linearen oder verzweigten Alkylrest, der 1 bis 6 Kohlenstoffatome zählt, noch einen Rest 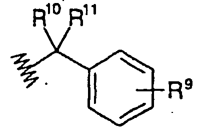 darstellt, bei dem R⁹ von 1 bis 3 Substituenten darstellt, die unabhängig voneinander ausgewählt sind aus einem Wasserstoffatom, einem Halogenatom und einem linearen oder verzweigten Alky- oder Alkoxyrest, der 1 bis 6 Kohlenstoffatome zählt, und bei dem R¹⁰ und R¹¹ unabhängig voneinander ausgewählt sind aus einem Wasserstoffatom und einem linearen oder verzweigten Alkylrest, der 1 bis 6 Kohlenstoffatome zählt;
- wenn R² einen nicht substituierten Biphenylrest darstellt oder wenn R² einen Phenylrest darstellt, der gegebenenfalls 1- bis 3-fach substituiert ist mit Substituenten, die unabhängig voneinander ausgewählt sind aus einem Halogenatom und einem der linearen oder verzweigten Alkyl- oder Alkoxyreste, die 1 bis 4 Kohlenstoffatome zählen, dann R¹ nicht einen -(CH₂)ₙ-Z-Rest darstellt, in dem n eine ganze Zahl von 0 bis 3 ist und Z eine linearen oder verzweigten Alkylrest, der 1 bis 4 Kohlenstoffatome zählt, oder einen Phenyl- oder Naphthylrest darstellt, der gegebenenfalls mit 1 bis 3 Substiuenten substituiert ist, die ausgewählt sind aus der Gruppe bestehend aus einem Halogenatom und einem linearen oder verzweigten Alkyl- oder Alkoxyrest, der 1 bis 4 Kohlenstoffatome zählt;
oder ein Salz dieser Verbindungen.

12. Verbindung nach Anspruch 11, **dadurch gekennzeichnet, dass** es aus folgenden Verbindungen ausgewählt ist:
- 2-(1-Pentylhexyl)-4-phenyl-1H-imidazol;
- 4-(4-Fluorphenyl)-2-(1-pentylhexyl)-1H-imidazol;
- 2-(Cyclohexylmethyl)-4-(4-fluorphenyl)-1H-imidazol;
- 4-(1,1'-Biphenyl-4-yl)-2-(1-phentylhexyl)-1H-imidazol;
- 4-(4-tert-Butylphenyl)-2-(1-propylbutyl)-1H-imidazol;
- 4-(4-Fluorphenyl)-2-(1-propylbutyl)-1H-imidazol;
- 4-(4'-Brom-1,1'-biphenyl-4-yl)-2-(1-propylbutyl)-1H-imidazol;
- 4-(1,1'-Biphenyl-4-yl)-2-(2cyclohexylethyl-1H-imidazol;
- 4-(1,1'-Biphenyl-4-yl)-2-[1-(4-isobutylphenyl)ethyl]-1H-imidazol;
- 4-(1,1'-Biphenyl-4-yl)-2-(1-phenylethyl)-1H-imidazol;
- 4-(1,1'-Biphenyl-4-yl)-2-(1-propylbutyl)-1H-imidazol;
- 4-(1,1'-Biphenyl-4-yl)-2-(cyclohexylmethyl)-1H-imidazol;
- 4-(1,1'-Biphenyl-4-yl)-2-(3-cyclohexlypropyl)-1H-imidazol;
und den Salzen dieser Verbindungen.

13. Verbindung der allgemeinen Formel (I), wie sie in Anspruch 1 definiert ist, oder ein Salz dieser Verbindung, **dadurch gekennzeichnet, dass** sie ausgewählt ist aus:
- 4-(1,1'-Biphenyl-4-yl)-2-(4-isobutylbenzyl)-1H-imidazol;
- 2-Hexyl-4-(4-isobutylphenyl)-1H-imidazol;
- 4-Phenyl-2-(3-phenylpropyl)-1H-imidazol;
- 4-(1,1'-Biphenyl-4-yl)-2-hexyl-1H-imidazol;
- 4-(4-Fluorphenyl)-2-[1-(4-isobutylphenyl)ethyl]-1H-imidazol;
und den Salzen dieser Verbindungen.

## Claims

1. Use of a compound of general formula **(I)** 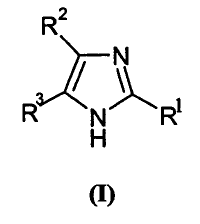 in which
R¹ represents a linear or branched alkyl radical containing from 3 to 16 carbon atoms or a cycloalkylalkyl radical the cycloalkyl radical of which contains from 3 to 7 carbon atoms and the alkyl radical of which is linear or branched and contains from 1 to 6 carbon atoms,
or also R¹ represents an aralkyl radical optionally substituted from 1 to 3 times on the aryl group by substituents chosen independently from a halogen atom and a linear or branched alkyl or alkoxy radical containing from 1 to 6 carbon atoms;
R² represents a phenyl radical optionally substituted from 1 to 3 times by substituents chosen independently from a halogen atom and a linear or branched alkyl or alkoxy radical containing from 1 to 6 carbon atoms,
or also R² represents a biphenyl radical optionally substituted from 1 to 4 times by one or more substituents chosen independently from a halogen atom and a linear or branched alkyl or alkoxy radical containing from 1 to 6 carbon atoms;
R³ represents a hydrogen atom or a linear or branched alkyl radical and containing from 1 to 6 carbon atoms;
or a pharmaceutically acceptable salt of a compound of general formula **(I)**
for preparing a medicament intended for the treatment or prevention of pain, the treatment of epilepsy, the treatment of cardiac rhythm disorders, the treatment of cerebrovascular accidents, the treatment of brain traumas, the treatment of neurodegenerative diseases, the treatment of depression and bipolar disorders, or the treatment of irritable bowel syndrome.

2. Use according to claim 1, **characterized in that** the medicament prepared is intended for the treatment or prevention of pain, the treatment of epilepsy, the treatment of cardiac rhythm disorders, the treatment of depression and bipolar disorders, or the treatment of irritable bowel syndrome.

3. Use according to claim 2, **characterized in that** the medicament prepared is intended for the treatment or prevention of pain or epilepsy.

4. Use according to one of claims 1 to 3, **characterized in that** the compound used is one of the following compounds:
- 4-(1,1'-biphenyl-4-yl)-2-(4-isobutylbenzyl)-1*H*-imidazole;
- 2-hexyl-4-(4-isobutylphenyl)-1H-imidazole;
- 4-phenyl-2-(3-phenylpropyl)-1H-imidazole;
- 2-(1-pentylhexyl)-4-phenyl-1H-imidazole;
- 4-(4-fluorophenyl)-2-(1-pentylhexyl)-1H-imidazole;
- 2-(cyclohexylmethyl)-4-(4-fluorophenyl)-1H-imidazole;
- 4-(1,1'-biphenyl-4-yl)-2-(1-pentylhexyl)-1H-imidazole;
- 4-(4-tert-butylphenyl)-2-(1-propylbutyl)-1H-imidazole;
- 4-(4-fluorophenyl)-2-(1-propylbutyl)-1H-imidazole;
- 4-(4'-bromo-1,1'-biphenyl-4-yl)-2-(1-propylbutyl)-1H-imidazole;
- 4-(1,1'-biphenyl-4-yl)-2-(2-cyclohexylethyl)-1H-imidazole;
- 4-(1,1'-biphenyl-4-yl)-2-hexyl-1H-imidazole;
- 4-(1,1'-biphenyl-4-yl)-2-[1-(4-isobutylphenyl)ethyl]-1H-imidazole;
- 4-(4-fluorophenyl)-2-[1-(4-isobutylphenyl)ethyl]-1H-imidazole;
- 4-(1,1'-biphenyl-4-yl)-2-(1-phenylethyl)-1H-imidazole;
- 4-(1,1'-biphenyl-4-yl)-2-(1-propylbutyl)-1H-imidazole;
- 4-(1,1'-biphenyl-4-yl)-2-(cyclohexylmethyl)-1H-imidazole;
- 4-(1,1'-biphenyl-4-yl)-2-(3-cyclohexylpropyl)-1H-imidazole;
or a pharmaceutically acceptable salt of one of the latter.

5. Use according to claim 4, **characterized in that** compound used is one of the following compounds:
- 4-(1,1'-biphenyl-4-yl)-2-(4-isobutylbenzyl)-1*H*-imidazole;
- 2-hexyl-4-(4-isobutylphenyl)-1H-imidazole;
- 2-(1-pentylhexyl)-4-phenyl-1H-imidazole;
- 4-(4-fluorophenyl)-2-(1-pentylhexyl)-1H-imidazole;
- 4-(1,1'-biphenyl-4-yl)-2-(1-pentylhexyl)-1H-imidazole;
- 4-(4-tert-butylphenyl)-2-(1-propylbutyl)-1H-imidazole;
- 4-(1,1'-biphenyl-4-yl)-2-(2-cyclohexylethyl)-1H-imidazole;
- 4-(1,1'-biphenyl-4-yl)-2-hexyl-1H-imidazole;
- 4-(1,1'-biphenyl-4-yl)-2-[1-(4-isobutylphenyl)ethyl]-1H-imidazole;
- 4-(1,1'-biphenyl-4-yl)-2-(1-propylbutyl)-1H-imidazole;
- 4-(1,1'-biphenyl-4-yl)-2-(cyclohexylmethyl)-1H-imidazole;
- 4-(1,1'-biphenyl-4-yl)-2-(3-cyclohexylpropyl)-1H-imidazole;
or a pharmaceutically acceptable salt of one of the latter.

6. As a medicament, a compound of general formula **(II)** 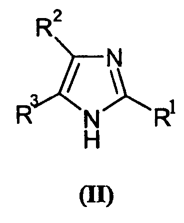 in which
R¹ represents a linear or branched alkyl radical containing from 3 to 16 carbon atoms or a cycloalkylalkyl radical the cycloalkyl radical of which contains from 3 to 7 carbon atoms and the alkyl radical of which is linear or branched and contains from 1 to 6 carbon atoms,
or also R¹ represents an aralkyl radical optionally substituted from 1 to 3 times on the aryl group by substituents chosen independently from a halogen atom and a linear or branched alkyl or alkoxy radical containing from 1 to 6 carbon atoms;
R² represents a phenyl radical optionally substituted from 1 to 3 times by substituents chosen independently from a halogen atom and a linear or branched alkyl or alkoxy radical containing from 1 to 6 carbon atoms,
or also R² represents a biphenyl radical optionally substituted from 1 to 4 times by one or more substituents chosen independently from a halogen atom and a linear or branched alkyl or alkoxy radical containing from 1 to 6 carbon atoms;
R³ represents a hydrogen atom or a linear or branched alkyl radical and containing from 1 to 6 carbon atoms;
it being understood however that:
- when R² represents a phenyl radical optionally substituted from 1 to 3 times by substituents chosen independently from a halogen atom and an alkyl or alkoxy radical, then R¹ represents neither a linear or branched alkyl radical containing from 1 to 6 carbon atoms nor a 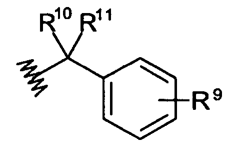 radical, in which R⁹ represents from one to three substituents chosen independently from a hydrogen atom, a halogen atom and a linear or branched alkyl or alkoxy radical containing from 1 to 6 carbon atoms, and R¹⁰ and R¹¹ being chosen independently from a hydrogen atom and a linear or branched alkyl radical containing from 1 to 6 carbon atoms;
- when R² represents a non-substituted biphenyl radical or when R² represents a phenyl radical optionally substituted from 1 to 3 times by substituents chosen independently from a halogen atom and one of the linear or branched alkyl or alkoxy radicals containing from 1 to 4 carbon atoms, then R¹ represents neither a -(CH₂)ₙ-Z radical in which n is an integer from 0 to 3 and Z represents a linear or branched alkyl radical containing from 1 to 4 carbon atoms or a phenyl or naphthyl radical optionally substituted by 1 to 3 substituents chosen from the group constituted by a halogen atom and the linear or branched alkyl or alkoxy radicals containing from 1 to 4 carbon atoms;
or a pharmaceutically acceptable salt of a compound of general formula **(II).**

7. Medicament according to claim 6, **characterized in that** it is one of the following compounds:
- 2-(1-pentylhexyl)-4-phenyl-1H-imidazole;
- 4-(4-fluorophenyl)-2-(1-pentylhexyl)-1H-imidazole;
- 2-(cyclohexylmethyl)-4-(4-fluorophenyl)-1H-imidazole;
- 4-(1,1'-biphenyl-4-yl)-2-(1-pentylhexyl)-1H-imidazole;
- 4-(4-tert-butylphenyl)-2-(1-propylbutyl)-1H-imidazole;
- 4-(4-fluorophenyl)-2-(1-propylbutyl)-1H-imidazole;
- 4-(4'-bromo-1,1'-biphenyl-4-yl)-2-(1-propylbutyl)-1H-imidazole;
- 4-(1,1'-biphenyl-4-yl)-2-(2-cyclohexylethyl)-1H-imidazole;
- 4-(1,1'-biphenyl-4-yl)-2-[1-(4-isobutylphenyl)ethyl]-1H-imidazole;
- 4-(1,1'-biphenyl-4-yl)-2-(1-phenylethyl)-1H-imidazole;
- 4-(1,1'-biphenyl-4-yl)-2-(1-propylbutyl)-1H-imidazole;
- 4-(1,1'-biphenyl-4-yl)-2-(cyclohexylmethyl)-1H-imidazole;
- 4-(1,1'-biphenyl-4-yl)-2-(3-cyclohexylpropyl)-1H-imidazole;
or a pharmaceutically acceptable salt of one of the latter.

8. As a medicament, a compound of general formula **(I)** as defined in claim 1, or its pharmaceutically acceptable salt, **characterized in that** it is chosen from:
- 4-(1,1'-biphenyl-4-yl)-2-(4-isobutylbenzyl)-1*H*-imidazole;
- 2-hexyl-4-(4-isobutylphenyl)-1H-imidazole;
- 4-phenyl-2-(3-phenylpropyl)-1H-imidazole;
- 4-(1,1'-biphenyl-4-yl)-2-hexyl-1H-imidazole;
- 4-(4-fluorophenyl)-2-[1-(4-isobutylphenyl)ethyl]-1H-imidazole;
and the pharmaceutically acceptable salts of the latter.

9. Pharmaceutical composition comprising, as active ingredient, a compound of general formula **(II)** as defined in claim 6, or a pharmaceutically acceptable salt of such a compound.

10. Pharmaceutical composition comprising, as active ingredient, a compound of general formula **(I)** as defined in claim 1, or a pharmaceutically acceptable salt of such a compound **characterized in that** it is chosen from:
- 4-(1,1'-biphenyl-4-yl)-2-(4-isobutylbenzyl)-1*H*-imidazole;
- 2-hexyl-4-(4-isobutylphenyl)-1H-imidazole;
- 4-phenyl-2-(3-phenylpropyl)-1H-imidazole;
- 4-(1,1'-biphenyl-4-yl)-2-hexyl-1H-imidazole;
- 4-(4-fluorophenyl)-2-[1-(4-isobutylphenyl)ethyl]-1H-imidazole;
and the pharmaceutically acceptable salts of the latter.

11. Compound of general formula **(III)** 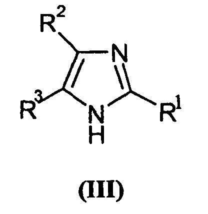 in which
R¹ represents a linear or branched alkyl radical containing from 3 to 16 carbon atoms or a cycloalkylalkyl radical the cycloalkyl radical of which contains from 3 to 7 carbon atoms and the alkyl radical of which is linear or branched and contains from 1 to 6 carbon atoms,
or also R¹ represents an aralkyl radical optionally substituted from 1 to 3 times on the aryl group by substituents chosen independently from a halogen atom and a linear or branched alkyl or alkoxy radical containing from 1 to 6 carbon atoms;
R² represents a phenyl radical optionally substituted from 1 to 3 times by substituents chosen independently from a halogen atom and a linear or branched alkyl or alkoxy radical containing from 1 to 6 carbon atoms,
or also R² represents a biphenyl radical optionally substituted from 1 to 4 times by one or more substituents chosen independently from a halogen atom and a linear or branched alkyl or alkoxy radical containing from 1 to 6 carbon atoms;
R³ represents a hydrogen atom or a linear or branched alkyl radical and containing from 1 to 6 carbon atoms;
it being understood however that:
- when R² represents a phenyl radical optionally substituted from 1 to 3 times by substituents chosen independently from a halogen atom and an alkyl or alkoxy radical, then R¹ represents neither a linear or branched alkyl radical containing from 1 to 6 carbon atoms nor a 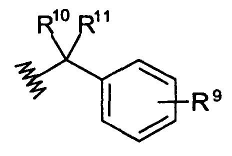 radical, in which R⁹ represents from one to three substituents chosen independently from a hydrogen atom, a halogen atom and a linear or branched alkyl or alkoxy radical containing from 1 to 6 carbon atoms, and R¹⁰ and R¹¹ being chosen independently from a hydrogen atom and a linear or branched alkyl radical containing from 1 to 6 carbon atoms;
- when R² represents a non-substituted biphenyl radical or when R² represents a phenyl radical optionally substituted from 1 to 3 times by substituents chosen independently from a halogen atom and one of the linear or branched alkyl or alkoxy radicals containing from 1 to 4 carbon atoms, then R¹ represents neither a -(CH₂)ₙ-Z radical in which n is an integer from 0 to 3 and Z represents a linear or branched alkyl radical containing from 1 to 4 carbon atoms or a phenyl or naphthyl radical optionally substituted by 1 to 3 substituents chosen from the group constituted by a halogen atom and the linear or branched alkyl or alkoxy radicals containing from 1 to 4 carbon atoms;
or a salt of one of these compounds.

12. Compound according to claim 11, **characterized in that** it is chosen from the following compounds:
- 2-(1-pentylhexyl)-4-phenyl-1H-imidazole;
- 4-(4-fluorophenyl)-2-(1-pentylhexyl)-1H-imidazole;
- 2-(cyclohexylmethyl)-4-(4-fluorophenyl)-1H-imidazole;
- 4-(1,1'-biphenyl-4-yl)-2-(1-pentylhexyl)-1H-imidazole;
- 4-(4-tert-butylphenyl)-2-(1-propylbutyl)-1H-imidazole;
- 4-(4-fluorophenyl)-2-(1-propylbutyl)-1H-imidazole;
- 4-(4'-bromo-1,1'-biphenyl-4-yl)-2-(1-propylbutyl)-1H-imidazole;
- 4-(1,1'-biphenyl-4-yl)-2-(2-cyclohexylethyl)-1H-imidazole;
- 4-(1,1'-biphenyl-4-yl)-2-[1-(4-isobutylphenyl)ethyl]-1H-imidazole;
- 4-(1,1'-biphenyl-4-yl)-2-(1-phenylethyl)-1H-imidazole;
- 4-(1,1'-biphenyl-4-yl)-2-(1-propylbutyl)-1H-imidazole;
- 4-(1,1'-biphenyl-4-yl)-2-(cyclohexylmethyl)-1H-imidazole;
- 4-(1,1'-biphenyl-4-yl)-2-(3-cyclohexylpropyl)-1H-imidazole;
and the salts of these compounds.

13. Compound of general formula **(I)** as defined in claim 1, or salt of such a compound, **characterized in that** it is chosen from:
- 4-(1,1'-biphenyl-4-yl)-2-(4-isobutylbenzyl)-1*H*-imidazole;
- 2-hexyl-4-(4-isobutylphenyl)-1H-imidazole;
- 4-phenyl-2-(3-phenylpropyl)-1H-imidazole;
- 4-(1,1'-biphenyl-4-yl)-2-hexyl-1H-imidazole;
- 4-(4-fluorophenyl)-2-[1-(4-isobutylphenyl)ethyl]-1H-imidazole;
and the salts of the latter.
